(19)

Europäisches
Patentamt

European
Patent Office

Office européen
des brevets

(11) **EP 3 357 481 B1**

(12) **EUROPEAN PATENT SPECIFICATION**

(45) Date of publication and mention
of the grant of the patent:
**26.06.2024 Bulletin 2024/26**

(21) Application number: **16852030.2**

(22) Date of filing: **26.09.2016**

(51) International Patent Classification (IPC):
*A61K 8/11* ^(2006.01)      *A61K 8/73* ^(2006.01)
*A61K 8/34* ^(2006.01)      *A61Q 19/00* ^(2006.01)
*A61K 8/81* ^(2006.01)

(52) Cooperative Patent Classification (CPC):
**A61K 8/11; A61K 8/34; A61K 8/342; A61K 8/731;
A61K 8/8152; A61Q 19/00; B01J 13/02;**
A61K 2800/10; A61K 2800/56; A61K 2800/652

(86) International application number:
**PCT/KR2016/010769**

(87) International publication number:
**WO 2017/057884 (06.04.2017 Gazette 2017/14)**

(54) **SELF-RESTORABLE CORE-SHELL CAPSULE**

SELBST-WIEDERHERSTELLBARE KERN-SCHALE-KAPSEL

CAPSULE À NOYAU-ENVELOPPE AUTO-RESTAURABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2015 KR 20150137589
23.09.2016 KR 20160122122**

(43) Date of publication of application:
**08.08.2018 Bulletin 2018/32**

(73) Proprietor: **Amorepacific Corporation
Seoul 04386 (KR)**

(72) Inventors:
• **SHIN, Ji Sik
Yongin-si
Gyeonggi-do 17074 (KR)**
• **CHO, Young Suk
Yongin-si
Gyeonggi-do 17074 (KR)**
• **PARK, Seung Han
Yongin-si
Gyeonggi-do 17074 (KR)**
• **CHAE, Byung Geun
Yongin-si
Gyeonggi-do 17074 (KR)**

(74) Representative: **Kraus & Lederer PartGmbB
Thomas-Wimmer-Ring 15
80539 München (DE)**

(56) References cited:
KR-A- 20040 084 364    KR-A- 20070 017 128
KR-B1- 101 168 038    KR-B1- 950 008 517
US-A1- 2006 292 193    US-A1- 2014 227 329

• BLAISZJK, B. J. ET AL.: 'Self-healing Polymers
and Composites' ANNUAL REVIEW OF
MATERIALS RESEARCH vol. 40, 2010, pages 179
- 211, XP055160115
• KOH, EUN JOO ET AL.: 'Polyurethane
Microcapsules for Self-healing Paint Coatings'
RSC ADVANCES vol. 4, 2014, pages 16214 -
16223, XP055386822
• YUAN, Y. C. ET AL.: 'Self Healing in Polymers and
Polymer Composites. Concepts, Realization and
Outlook: A Review' EXPRESS POLYMER
LETTERS vol. 2, no. 4, 2008, pages 238 - 250,
XP055386826

# Description

[Technical Field]

The present invention relates to a self-healable core-shell capsule.

[Background Art]

Self-healing means that materials such as polymers damaged by an external environment may detect defects by themselves to restore their structure, and also to recover the original function, and since the damage of the materials causes loss of physical properties and function of the materials themselves, and has a great impact on durability and performance persistence of an entire product, there is a rapidly growing interest in development of self-healing materials allowing fundamental restoration of structure and function of materials as well as suppressing damage.

As one of the self-healable materials, S. R. White, etc., introduced a self-healing system healing damage of thermosetting materials, using microcapsules containing monomers (Non-patent document 1). This self-healing system has a form in which liquid phase monomers are encapsulated, and these capsules are present to be dispersed in matrix materials together with catalyst particles, and in the case that microcapsules in the matrix are damaged by an external impact and the like, the monomers contained in the capsules flow out to be polymerized in the presence of the catalyst in the matrix, so that the capsules and matrix may be restored by themselves.

However, in this case, there are fundamental limitations in that the catalyst to be used is expensive, physical property resilience reaches only about 75% due to interfacial heterogeneity at the healing site, and most of all, a portion of once healed is not available for re-healing.

(Non-patent document 1) S. R. White, N. R. Sottos, P. H. Geubelle, J. S. Moore, M. R. Kessler, S. R. Sriram, E. N. Brown, and S. Viswanathan, Nature, 409, 794 (2001).

Among further prior art, KR 10-1168038 B1 discloses a microcapsule including a core unit and polymer, and a capsule film that surrounds the core unit.

## [Disclosure]

[Technical Problem]

The present invention was made for solving the above problems, and an object of the present invention is to provide a core-shell capsule having excellent self-healing capability to restore the shape of the capsule, when a physical crack occurs in the capsule.

[Technical Solution]

The present invention relates to a core-shell capsule according to claim 1.

In another general aspect, a cosmetic composition includes the core-shell capsules.

[Advantageous Effects]

The core-shell capsule according to the present invention has a merit of restoring the original form by itself to maintain a stable capsule shape, when a physical crack occurs in the capsule by an external impact, severe temperature change, or the like.

In addition, as the capsule allows self-healing, the core material inside of the capsule may not be influenced, such as degenerated by an external environment, and thus, the present invention has a merit of maintaining the efficacy of the core for a long time.

[Description of Drawings]

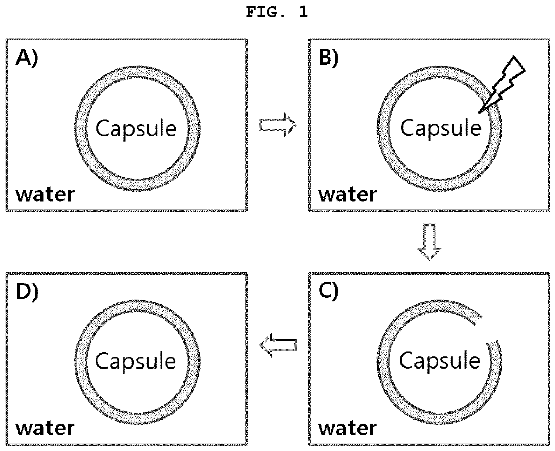

FIG. 1 illustrates self-healing capacity of a core-shell capsule according to an exemplary embodiment of the present invention, in which A) represents an initial core-shell capsule, B) represents that the capsule is subjected to an external impact, C) represents that the shell is damaged, and D) represents self-healing of the shell by effluence of an internal material.

FIG. 2 is a photograph of core-shell capsules prepared according to Example 1 and Comparative Example 1, in which self-healing of the capsules when an external impact was applied is compared.

FIG. 3 is a photograph of core-shell capsules prepared according to Example 1 and Comparative Example 2, in which self-healing of the capsules when severe temperature change was applied is compared.

[Best Mode]

Hereinafter, the core-shell capsule according to the present invention will be described in detail with reference to the accompanying drawings. The drawings to be provided below are provided by way of example so that the idea of the present invention can be sufficiently transferred to a person skilled in the art to which the present invention pertains. The drawings suggested below will be exaggerated in order to clear the spirit and scope of the present invention. In addition, like reference numerals denote like elements throughout the specification.

Technical terms and scientific terms used herein have the general meaning understood by those skilled in the art to which the present invention pertains unless otherwise defined, and a description for the known function and configuration unnecessary obscuring the gist of the present invention will be omitted in the following description and the accompanying drawings.

The present invention relates to a core-shell capsule which may restore the original form by itself to maintain a stable capsule shape, when a physical crack occurs in the capsule by an external impact, severe temperature change, or the like.

More specifically, in the core, a cellulose-based polymer compound which is a raw material of a polymer shell remains in a dissolved state, and when a crack occurs in the polymer shell, the cellulose-based polymer compound dissolved in the core is partially precipitated in the cracked portion of the shell by an external environment to restore the cracked portion of the shell, thereby self-healing the capsule shape. Accordingly, the capsule may be self-healed by itself even in the case of the damage by an external impact, severe temperature change, or the like, and thus, may have very high stability. In addition, since self-healing is possible, the core material inside of the capsule may not be influenced such as degenerated by an external environment, thereby maintaining the efficacy of the core for a long time, and since an oil which is one of the core materials may be stably positioned inside of each capsule, the oil is prevented from coalescing, so that it is not easily phase-separated. As another merit of the self-healing capability, a damaged capsule is self-healed to maintain its original form, thereby having an excellent appearance, and the maintenance of a capsule shape provides a unique sense of use.

Specifically, according to claim 1, the core-shell capsule according to an exemplary embodiment of the present invention includes: a core including a synthetic oil, an alcohol-based solvent satisfying the following Equation 1, and a cellulose-based polymer compound dissolved in the alcohol-based solvent; and a water-insoluble polymer shell surrounding the core:

$$[\text{Equation 1}]$$

$$0.01 \geq C_A/C_B \geq 100$$

wherein $C_A/C_B$ is a distribution coefficient of the alcohol-based solvent, in which when the alcohol-based solvent is dissolved in synthetic oil and water to reach equilibrium, $C_A$ is a concentration of the alcohol-based solvent dissolved in the synthetic oil, and $C_B$ is a concentration of the alcohol-based solvent dissolved in the water.

For enabling the self-healing, an alcohol-based solvent which dissolves the water-insoluble polymer compound well, and satisfies Equation 1 should be well selected. Equation 1 relates to a distribution coefficient of a solvent, and when a crack occurs in the polymer shell, the alcohol-based solvent according to the present invention has the distribution coefficient satisfying Equation 1, so that it may be diffused to the external phase such as water, and the cellulose-based polymer compound dissolved in the solvent is diffused together with the diffused solvent to flow out to the outside. Here, the cellulose-based polymer compound flowing out to the outside of the core may be precipitated by an external environment such as water to restore the cracked part of the polymer shell. In addition, by using the alcohol-based solvent satisfying Equation 1, a hard polymer shell may be formed, when forming the capsule, and a capsule having high stability may be prepared.

However, when the distribution coefficient of the solvent is more than 100, the cellulose-based polymer compound dissolved in the core is difficult to flow out to the outside of the core, so that the capsule is not well formed, or self-healing is not well done, and when the distribution coefficient is less than 0.01, the alcohol-based solvent and the cellulose-based polymer compound dissolved in the solvent all exit to the external phase such as water, before shell restoration, or shell restoration is possible, but the alcohol-based solvent and the cellulose-based polymer compound dissolved in the solvent all exit to the external phase such as water, so that the self-healing capability is limited to once.

Preferably, the solvent according to an exemplary embodiment may satisfy the following Equation 1-1:

$$[\text{Equation 1-1}]$$

$$0.1 \geq C_A/C_B \geq 10$$

wherein $C_A/C_B$ is a distribution coefficient of the alcohol-based solvent, in which when the alcohol-based solvent is dissolved in synthetic oil and water to reach equilibrium, $C_A$ is a concentration of the alcohol-based solvent dissolved in the oil, and $C_B$ is a concentration of the alcohol-based solvent dissolved in the water.

By using the alcohol-based solvent having the distribution coefficient satisfying the above range, the present invention has a merit in that the alcohol-based solvent and the cellulose-based polymer compound dissolved in the alcohol-based solvent are diffused to the external phase when the capsule is damaged, thereby recovering the damaged part of the capsule more effectively, and also the diffusion to the outside is not so rapid, so that the self-healing capability is not limited to once, but may be maintained for several times to several hundred times. The distribution coefficient of the alcohol-based solvent may be more preferably $C_A/C_B$ 0.1 to 3, particularly preferably 0.2 to 1.

According to the present invention, the solvent is an alcohol-based solvent. Specifically, the alcohol-based solvent is ethanol. In the present invention, since the core-shell capsule may be applied as a cosmetic composition, the alcohol-based solvent is ethanol.

In addition, the alcohol-based solvent according to the present invention has a boiling point of 50 °C or more. When the boiling point is less than 50 °C which is unduly low, the solvent in the capsule is easily evaporated, so that the water-insoluble polymer compound may be precipitated inside of the capsule, and as the solvent is not diffused to the outside of the capsule even in the case that a crack occurs in the polymer shell, the cellulose-based polymer compound is difficult to be in contact with an external phase such as water, etc. in the cracked part, and thus, the polymer shell may be difficult to be formed. The upper limit of the boiling point is not particularly limited.

The cellulose-based polymer compound according to an exemplary embodiment of the present invention is, as described above, dissolved in the core which is an internal phase, and flows from the inside to the outside of the capsule through the cracked part of the shell, so as to be precipitated as a polymer material by an external phase such as water, when the capsule is damaged, thereby restoring the damaged capsule film (polymer shell). Accordingly, it is preferred to select the cellulose-based polymer compound which is easily dissolved in the alcohol-based solvent, and not dissolved in an external phase such as water.

According to the invention, the cellulose-based polymer compound is ethyl cellulose.

In addition, in the present invention, in order for the core-shell capsule to have self-healing capability, it is necessary to properly adjust the contents of the cellulose-based polymer compound and the alcohol-based solvent in the core. According to the invention, the weight ratio of the cellulose-based polymer compound : alcohol-based solvent in the core is 1:0.5 to 100. Within this range, the core-shell capsule may have excellent self-healing capability. Preferably the weight ratio of the cellulose-based polymer compound : alcohol-based solvent may be 1:1 to 50, more preferably the weight ratio of the cellulose-based polymer compound : alcohol-based solvent may be 1:3 to 10. When the amount of the cellulose-based polymer compound relative to the alcohol-based solvent is unduly small, even in the case that the alcohol-based solvent and the cellulose-based polymer compound dissolved in the alcohol-based solvent flow out to the external phase by damage of the capsule, the amount of the precipitated cellulose-based polymer compound is insignificant, so that the restoration of the damaged shell may be difficult, and when the amount of the cellulose-based polymer compound relative to the alcohol-based solvent is unduly large, the cellulose-based polymer compound may not be completely dissolved in the solvent, and the solvent may be partially diffused when the capsule is formed, thereby making the formation of the shell difficult.

The synthetic oil according to the present invention is octyl dodecanol.

In addition, in the present invention, in order for the core-shell capsule to have self-healing capability, it is necessary to properly adjust the content of the synthetic oil in the core. When the amount of the synthetic oil is unduly small, the solvent diffusion rate to the external phase may be too high, and when the amount of the synthetic oil is unduly large, the content of the alcohol-based solvent is lowered so that the solubility of the cellulose-based polymer compound may be decreased. According to the invention, the weight ratio of the alcohol-based solvent : the synthetic oil in the core is 1:0.1 to 10. Within this range, the solvent is diffused at an appropriate rate, so that the core-shell capsule may have excellent self-healing capability, and the water-insoluble polymer compound may be well dissolved in the solvent. Preferably the weight ratio of the alcohol-based solvent : the synthetic oil may be preferably 1:0.5 to 5, more preferably the weight ratio of the alcohol-based solvent : the synthetic oil may be 1:0.8 to 2. Within this range, the cellulose-based polymer compound may be easily dissolved, and the diffusion rate of the alcohol-based solvent is properly adjusted, so that the alcohol-based solvent and the cellulose-based polymer compound dissolved in the solvent may be diffused to the external phase when the capsule is damaged, thereby self-healing the damaged part of the capsule more effectively.

Meanwhile, in the present invention, the core-shell capsule may have an average diameter of 0.1 to 10 mm, more preferably 0.5 to 5 mm, still more preferably 0.5 to 3 mm. Within this range, the capsule may be used as a formulation more easily.

In the present invention, the shell in the core-shell capsule may have a thickness of 1 nm to 3 mm, more preferably 2 nm to 2 mm, still more preferably 2 nm to 1 mm. Within this range, the capsule may not be easily broken with small impact, and a sense of use when applied on skin may be excellent.

In addition, another embodiment of the present invention relates to a cosmetic composition including the core-shell capsules as described above, and the cosmetic composition may include water which is an external phase together with the core-shell capsules.

Specifically, the present invention relates to a cosmetic composition including core-shell capsules as claimed, including: a core including a synthetic oil, an alcohol-based solvent satisfying the following Equation 1, and a cellulose-based polymer compound dissolved in the alcohol-based solvent; and a water-insoluble polymer shell surrounding the core, as an internal phase, and water as an external phase.

[Equation 1]

$$0.01 \geq C_A/C_B \geq 100$$

wherein $C_A/C_B$ is a distribution coefficient of the solvent, in which when the solvent is dissolved in oil and water to reach equilibrium, $C_A$ is a concentration of the solvent dissolved in the oil, and $C_B$ is a concentration of the solvent dissolved in the water.

As described above for the core-shell capsule, as the core-shell capsule has water as the external phase, when a crack occurs in the polymer shell, the cellulose-based polymer compound dissolved in the core flows out of the damaged part of the shell to water which is the external phase, and is partially precipitated by the water at this time to restore the cracked portion of the shell, thereby self-healing the capsule shape. Accordingly, the capsule may be self-healed by itself even in the case of damage by an external impact, severe temperature change, or the like, and thus, the cosmetic composition according to the present invention may have very high stability.

In an exemplary embodiment of the present invention, the cosmetic composition may further include surfactants, sunscreen agents, antioxidants, stabilizers, pH adjusting agents, preservatives, disinfectants, coloring agents, perfumes, pearling agents, which are commonly used in the art, depending on the purpose of the cosmetic composition, within the range of not deteriorating the self-healing capability of the core-shell capsule.

The cosmetic composition having such excellent self-healing capability may be used e.g. for skin, lotion, essence, cream, makeup base, foundation, concealer.

Hereinafter, the core-shell capsule and the cosmetic composition according to the present invention will be described in more detail by the following Examples. Unless otherwise defined, all technical terms and scientific terms have the same meanings as those commonly understood by a person skilled in the art to which the present invention pertain. The terms used herein is only for effectively describing a certain exemplary embodiment. In addition, the singular form used in the specification and claims appended thereto may be intended to also include a plural form, unless otherwise indicated in the context. Further, unless otherwise stated, the unit of added materials herein may be wt%.

[Example 1]

Core-shell capsules were prepared using fluidics.

Specifically, a mixed solution of 10 wt% of ethyl cellulose, 44.9 wt% of ethanol, 45 wt% of octyl dodecanol and 0.1 wt% of lithospermum erythrorhizon root extract, as an internal phase material was dispersed in water (containing 0.1 wt% of acrylate/C10-30 alkyl acrylate crosspolymer and 0.07 wt% of tromethamine) as an external phase, using fluidics, thereby preparing core-shell capsules having an average diameter of 1 mm. Here, the distribution coefficient of ethanol, $C_A/C_B$ was 0.41, and it was calculated by adding 30 g of water and octyldodecanol to two measuring cylinders, respectively, further adding 20 g of ethanol, respectively, mixing them, and measuring the volume increase ratio in the two measuring cylinders. The lithospermum erythrorhizon root extract as a coloring agent was added for easily observing release of the internal phase material and shape maintenance of the capsules.

[Comparative Example 1]

20 wt% of ethyl cellulose and 80 wt% of ethanol were mixed to prepare a mixed solution, from which a capsule precursor was prepared using the same manner as in Example 1, and the solvent was volatilized at high temperature to prepare empty hard capsules (only having a shell), and thereafter, the inside thereof was filled with a mixed solution of 99.9 wt% of ethanol and 0.1 wt% of corydalis incisa extract again, thereby preparing core-shell capsules.

[Comparative Example 2]

Core-shell capsules were prepared in the same manner as in Example 1, except for using dipropylene glycol instead of ethanol, and ferric ferrocyanide as the coloring agent. Here, almost all dipropylene glycol migrated to a water phase, so that the distribution coefficient of dipropylene glycol converged to almost 0.

[Comparative Example 3]

All processes proceeded in the same manner as in Example 1, except for using squalane instead of ethanol, and ferric ferrocyanide as the coloring agent, but core-shell capsules were not formed. Here, squalane hardly migrated to the water phase and remained in the oil phase, so that the distribution coefficient of squalane converged to almost infinity.

[Physical property evaluation]

1) Repeated impact experiment

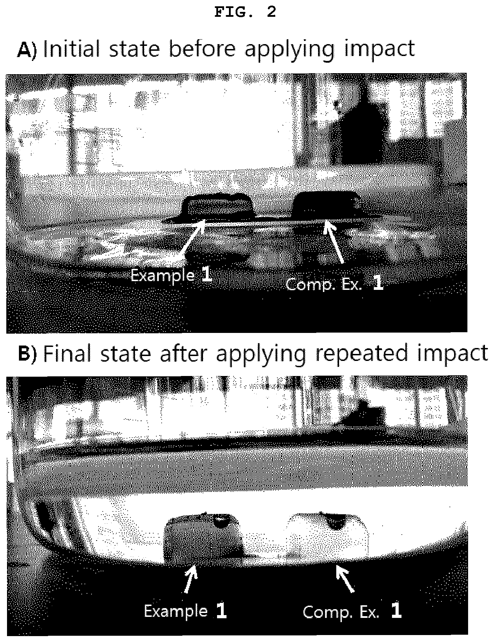

The core-shell capsules prepared from Example 1 and Comparative Example 1 were repeatedly impacted with a needle so that the capsules were damaged.
As a result, as illustrated in FIG. 2, it was confirmed in Example 1 that ethyl cellulose dissolved in ethanol was diffused in the portion of the shell cracked by repeated impact to the outside and precipitated, thereby recovering the cracked portion of the shell to maintain the capsule shape, and stably preserving the internal material.
However, it was confirmed in Comparative Example 1 that the shell was damaged by repeated impact, but since only ethanol was present as the internal material, the self-healing of the shell was impossible, and the internal material was all diffused to the outside.

2) Repeated freezing experiment

The core-shell capsules prepared in Example 1 and Comparative Example 2 were frozen at -20 °C, and then thawed at room temperature of about 25 °C, which is repeated three times.

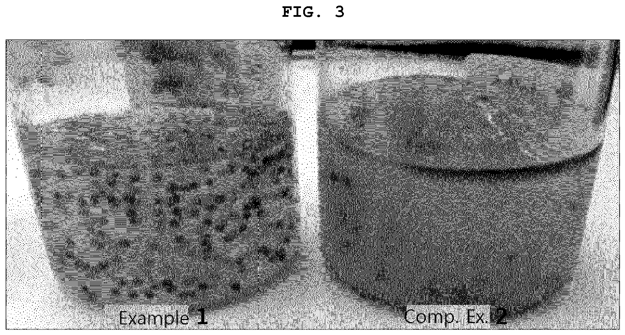

As a result, as illustrated in FIG. 3, it was confirmed in Example 1 that the internal phase material in the core-shell capsule was stably preserved, which shows that even in the case that the core-shell capsules were damaged by severe temperature change, the capsules were self-healed by themselves, so that the capsule shape was maintained very stably.

However, it was confirmed in Comparative Example 2 that since the capsule shape was destroyed by severe temperature change, the internal material flowed out to the outside.

## Claims

1. A core-shell capsule comprising:

   a core including a synthetic oil, an alcohol-based solvent satisfying the following Equation 1, and a water-insoluble cellulose-based polymer compound dissolved in the alcohol-based solvent; and
   a water-insoluble polymer shell surrounding the core:

$$[Equation\ 1]$$
$$0.01 \geq C_A/C_B \geq 100$$

   wherein $C_A/C_B$ is a distribution coefficient of the alcohol-based solvent, in which when the alcohol-based solvent is dissolved in synthetic oil and water to reach equilibrium, $C_A$ is a concentration of the alcohol-based solvent dissolved in the synthetic oil, and $C_B$ is a concentration of the alcohol-based solvent dissolved in the water,
   wherein a weight ratio of the cellulose-based polymer compound to the alcohol-based solvent in the core is 1:0.5 to 100,
   wherein a weight ratio of the alcohol-based solvent to the synthetic oil in the core is 1:0.1 to 10, **characterized in that** the synthetic oil is octyl dodecanol, the alcohol-based solvent is ethanol, the cellulose-based polymer compound is ethyl cellulose, and the water-insoluble polymer is acrylate/C10-30 alkyl acrylate crosspolymer.

2. The core-shell capsule of claim 1, wherein the core-shell capsule has an average diameter of 0.1 to 10 mm.

3. The core-shell capsule of claim 2, wherein the shell has a thickness of 1 nm to 3 mm.

4. A cosmetic composition comprising the core-shell capsule of any one of claims 1 to 3.

## Patentansprüche

1. Kern-Hülle-Kapsel, umfassend:

   einen Kern, der ein synthetisches Öl, ein Lösungsmittel auf Alkoholbasis, das die folgende Gleichung 1 erfüllt, und eine wasserunlösliche Polymerverbindung auf Zellulosebasis, die in dem Lösungsmittel auf Alkoholbasis gelöst ist, enthält; und
   eine wasserunlösliche Polymerhülle, die den Kern umgibt:

$$[Gleichung\ 1]$$
$$0,01 \leq C_A/C_B \leq 100$$

   wobei $C_A/C_B$ ein Verteilungskoeffizient des Lösungsmittels auf Alkoholbasis ist, wobei, wenn das Lösungsmittel auf Alkoholbasis in synthetischem Öl und Wasser gelöst wird, um ein Gleichgewicht zu erreichen, $C_A$ eine Konzentration des Lösungsmittels auf Alkoholbasis ist, die in dem synthetischen Öl gelöst ist, und $C_B$ eine Konzentration des Lösungsmittels auf Alkoholbasis ist, die in dem Wasser gelöst ist, wobei das Gewichtsverhältnis der Polymerverbindung auf Cellulosebasis zu dem Lösungsmittel auf Alkoholbasis im Kern 1:0,5 bis 100 beträgt, wobei das Gewichtsverhältnis zwischen dem Lösungsmittel auf Alkoholbasis und dem synthetischen Öl im Kern 1:0,1 bis 10 beträgt, **dadurch gekennzeichnet, dass** das synthetische Öl Octyldodecanol ist, das Lösungsmittel auf Alkoholbasis Ethanol ist, die Polymerverbindung auf Cellulosebasis Ethylcellulose ist und das wasserunlösliche Polymer Acrylat/C10-30-Alkylacrylat-Kreuzpolymer ist.

2. Kern-Hülle-Kapsel nach Anspruch 1, wobei die Kern-Hülle -Kapsel einen durchschnittlichen Durchmesser von 0,1 bis 10 mm hat.

3. Kern-Hülle-Kapsel nach Anspruch 2, wobei die Hülle eine Dicke von 1 nm bis 3 mm aufweist.

4. Kosmetische Zusammensetzung, umfassend die Kern-Hülle-Kapsel nach einem der Ansprüche 1 bis 3.

**Revendications**

1. Capsule noyau-coquille comprenant :

   un noyau comprenant une huile synthétique, un solvant à base d'alcool satisfaisant à l'équation 1 suivante, et
   un composé polymère à base de cellulose insoluble dans l'eau dissous dans le solvant à base d'alcool ; et
   une coquille de polymère insoluble dans l'eau entourant le noyau :

   [Équation 1]

   $$0{,}01 \leq C_A/C_B \leq 100$$

   où $C_A/C_B$ est un coefficient de distribution du solvant à base d'alcool, dans lequel, lorsque le solvant à base d'alcool est dissous dans l'huile synthétique et l'eau pour atteindre l'équilibre, $C_A$ est une concentration du solvant à base d'alcool dissous dans l'huile synthétique, et $C_B$ est une concentration du solvant à base d'alcool dissous dans l'eau,
   dans laquelle le rapport en poids entre le composé polymère à base de cellulose et le solvant à base d'alcool dans le noyau est de 1:0,5 à 100,
   dans laquelle le rapport en poids entre le solvant à base d'alcool et l'huile synthétique dans le noyau est de 1:0,1 à 10,
   **caractérisé en ce que** l'huile synthétique est le dodécanol d'octyle, le solvant à base d'alcool est l'éthanol, le composé polymère à base de cellulose est l'éthylcellulose et le polymère insoluble dans l'eau est le crosspolymère acrylate/alkyl acrylate C10-30.

2. Capsule noyau-coquille selon la revendication 1, dans laquelle la capsule noyau-coquille a un diamètre moyen de 0,1 à 10 mm.

3. Capsule noyau-coquille selon la revendication 2, dans laquelle la coquille a une épaisseur comprise entre 1 nm et 3 mm.

4. Composition cosmétique comprenant la capsule noyau-coquille selon l'une des revendications 1 à 3.

FIG. 1

FIG. 2

**A)** Initial state before applying impact

**B)** Final state after applying repeated impact

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101168038 B1

**Non-patent literature cited in the description**

- **S. R. WHITE ; N. R. SOTTOS ; P. H. GEUBELLE ; J. S. MOORE ; M. R. KESSLER ; S. R. SRIRAM ; E. N. BROWN ; S. VISWANATHAN.** *Nature,* 2001, vol. 409, 794